# EUROPÄISCHE PATENTSCHRIFT

(11) **EP 1 899 420 B1**
(45) Veröffentlichungstag und Bekanntmachung des Hinweises auf die Patenterteilung: **29.03.2017**
(21) Anmeldenummer: 06754407.2
(22) Anmeldetag: 16.06.2006
(51) Int. Cl.: C09C 1/00

(54) **PIGMENTE**
PIGMENTS
PIGMENTS

(30) Priorität: 29.06.2005 DE 102005030242
(43) Veröffentlichungstag der Anmeldung: 19.03.2008
(73) Patentinhaber: Merck Patent GmbH, 64293 Darmstadt (DE)
(72) Erfinder: KNIESS, Helge Bettina, 64380 Rossdorf (DE); PFAFF, Gerhard, 64839 Münster (DE)
(86) Internationale Anmeldenummer: PCT/EP2006/005802
(87) Internationale Veröffentlichungsnummer: WO 2007/000253

(56) Entgegenhaltungen:
- EP-A- 0 913 432
- EP-A2- 0 246 523
- EP-A2- 0 668 329
- US-A1- 2004 151 827

## Beschreibung

Die vorliegende Erfindung betrifft Pigmente umfassend ein Substrat und eine darauf aufgebrachte Eisen-haltige Beschichtung umfassend metallisches Eisen, ein Verfahren zur Herstellung der erfindungsgemäßen Pigmente und deren Verwendung.

Der Einsatz von Effektpigmenten in den unterschiedlichsten Anwendungen gewinnt zunehmende Bedeutung. Im Automobilbereich, bei der Einfärbung von Kunststoffen, in der Kosmetik aber auch im Druckbereich werden zunehmend Effektpigmente eingesetzt, die den damit pigmentierten Produkten einen besonderen Glanz oder besondere Farbeffekte verleihen sollen. In der Regel handelt es sich bei den Effektpigmenten um Substrate, z.B. aus Metallen, Glimmer oder synthetischen Plättchen aus SiO₂, Glas oder Al₂O₃, die mit einer oder mehreren Schichten, z.B. aus Metallen oder Metalloxiden beschichtet sind. Insbesondere Metalloxide sind häufig verwendete Schichtmaterialien, da sie durch Auffällung auf die Substrate aufgebracht werden können und chemisch weitestgehend inert sind. Ein gängiges eingesetztes Metalloxid ist Eisenoxid.

In US 2004/151827 A1 sind Plättchen mit einheitlicher Form und Dicke zur Verwendung als Tracer in Sicherheitsanwendungen beschrieben, wobei die Plättchen sowohl aus einer einzigen Schicht eines dielektrischen Materials, z.B. ZnS, bestehen können als auch aus Schichtmaterialien, die Metallsubstrate umfassen. Aus EP 0 668 329 A2 sind Metalleffektpigmente bekannt und EP 0 913 432 A1 betrifft beschichtete, ggf. metalbeschichtete, Partikel mit geringem Eigengewicht wie z. B. pulverförmige Kunststoffe und anorganische Hohlkugeln. Eisenoxid-haltige Effektpigmente sind bekannt und beispielsweise in EP 0 307 747 oder EP 0 246 523 beschrieben. Bei der Beschichtung von Substraten mit Eisen(III)-oxid werden Pigmente mit rotbrauner Körperfarbe erhalten. Ebenfalls bekannt sind Pigmente mit einer Beschichtung aus Eisen(II)-oxid, wobei entweder, je nach Anteil des Oxids, schwarze glanzlose Pigmente oder glänzende farbige Pigmente erhalten werden. Alternativ lassen sich schwarze Pigmente durch Beschichtung von Substraten mit Fe₃O₄ erhalten, wie sie in EP 1 520 883 beschrieben sind. Zusätzlich zu der gewünschten dunklen Körperfarbe können Eisenoxid-haltige Pigmente, je nach Art des verwendeten Eisenoxids auch funktionelle Eigenschaften aufweisen, beispielsweise magnetisierbar sein.

Die oben genannten Pigmente haben den Nachteil, dass sie entweder eine schwarze Körperfarbe oder einen hohen Glanz zeigen, aber nicht die durchaus wünschenswerte Kombination dieser Eigenschaften aufweisen. Gerade diese Kombination ist aber insbesondere bei Autolacken von besonderer Bedeutung, da es gerade für dunkel lackierte Fahrzeuge mit hohem Glanz großes Interesse von Autoherstellern und Kunden gibt. Weiterer Nachteil bei den bekannten technischen Lösungen ist, dass zur Erzeugung des gewünschten dunklen Farbtons und zur Erzeugung von Deckvermögen Ruß als Schwarzpigment zugegeben werden muss. Durch den absorbierenden und lichtstreuenden Ruß kommt es zu Glanzverlusten.

Bei den bekannten magnetisierbaren Eisenoxid-haltigen Pigmenten ist es außerdem nachteilig, dass diese Magnetisierbarkeit immer an eine dunkle Körperfarbe, nämlich dunkelbraun, grau oder schwarz gebunden ist.

Es besteht daher ein Bedarf an neuen Pigmenten, die bei neutraler Körperfarbe zusätzliche interessante optische Effekte sowie einen hohen Glanz aufweisen. Darüber hinaus besteht ein Bedarf an funktionellen, insbesondere magnetisierbaren Pigmenten, die über ein breites Farbspektrum verfügen und leicht magnetisierbar sind.

Gegenstand der vorliegenden Erfindung sind demgemäss Pigmente umfassend ein Substrat und eine metallisches Eisen enthaltende Beschichtung, wobei das Substrat Titanoxide, synthetischen oder natürlichen Glimmer, Schichtsilikate, Glas, SiO₂, Al₂O₃, Graphit, und/oder BiOCl umfasst, das metallische Eisen in der Eisen-haltigen Beschichtung in Kombination mit FeO und/oder Fe₃O₄ vorliegt und der Anteil an metallischen Eisen 30 bis 99 Gew.-% bezogen auf die Eisen-haltige Beschichtung beträgt.

Die erfindungsgemäßen Pigmente zeigen in einer ersten Ausführungsform einen hohen Glanz und sind dabei farbneutral, vorzugsweise grauschwarz.

Darüber hinaus zeigen sie eine edel anmutende, z.B. silberne oder goldfarbene, Interferenz. Darüber hinaus ist die metallisches Eisen enthaltende Beschichtung frei von Kohlenstoff oder anderen Verunreinigungen, die aus einer Reduktion mit Kohlenwasserstoffen oder Metallen, wie z.B. Lithium, Natrium, Calcium oder anderen Metallen,
stammen. In den Anwendungen der erfindungsgemäßen Pigmente kann zudem auf die zusätzliche Zugabe von Ruß zur Erzeugung bestimmter dunkler Farbtöne und/oder zur Erzeugung von Deckvermögen ganz oder teilweise verzichtet werden. Damit ist es möglich, in unterschiedlichen, dunklen Formulierungen Ruß teilweise oder vollständig durch das erfindungsgemäße Pigment zu ersetzen.

In einer zweiten Ausführungsform sind die erfindungsgemäßen Pigmente magnetisierbar, weisen eine grauschwarze oder bunte Körperfarbe auf und/oder zeigen bunte Interferenzfarbeffekte und besitzen gleichzeitig ein hohes Deckvermögen. Darüber hinaus können sie auch optional die so genannten Farb-Flopp-Effekte, also sich mit dem Beleuchtungs- und/oder Betrachtungswinkel ändernde Farbeffekte, aufweisen.

Ebenfalls Gegenstand der vorliegenden Erfindung ist ein Verfahren zur Herstellung der erfindungsgemäßen Interferenzpigmente, wobei ein gegebenenfalls zusätzlich mit einer oder mehreren Schichten beschichtetes Substrat, welches als äußerste Schicht eine Eisenoxid-haltige Beschichtung aufweist, in einem Gasgemisch aus Stickstoff und Wasserstoff unter Bildung von metallischem Eisen umgesetzt sowie gegebenenfalls mit einer oder mehreren weiteren Schichten beschichtet wird.

Die erfindungsgemäßen Pigmente sind vielseitig einsetzbar. Daher ist die Verwendung von Interferenzpigmenten gemäß der vorliegenden Erfindung in Kosmetika, Lacken, Farben, Kunststoffen, Folien, in Sicherheitsanwendungen, zur Lasermarkierung, zur Saatguteinfärbung, zur Lebensmitteleinfärbung oder in Arzneimittelüberzügen sowie zur Herstellung von Pigmentpräparationen und Trockenpräparaten ebenfalls Gegenstand der vorliegenden Erfindung.

Die erfindungsgemäßen Pigmente basieren auf Substraten, vorzugsweise auf plättchenförmigen Substraten, wobei das Substrat synthetischen oder natürlichen Glimmer, Schichtsilikate, Glas, Borosilikate, SiO₂, Al₂O₃, Titanoxide, bevorzugt TiO₂, Graphit, und/oder BiOCl umfasst. Insbesondere handelt es sich bei den Substraten um plättchenförmigen Glimmer, plättchenförmiges Glas, plättchenförmiges SiO₂ oder plättchenförmiges Al₂O₃.
Bei den erfindungsgemäßen Pigmenten können zwischen der metallisches Eisen enthaltenden Beschichtung und dem Substrat zusätzlich eine oder mehrere Schichten enthaltend Metalloxide, Metalloxidhydrate, Metallsuboxide, Metalle, Metallfluoride, Metallnitride, Metalloxynitride und/oder Mischungen hieraus vorliegen.
Die Metalloxid-, Metalloxidhydrat, Metallsuboxid-, Metall-, Metallfluorid-, Metallnitrid-, Metalloxynitridschichten oder die Mischungen hieraus können niedrig- (Brechzahl < 1.8) oder hochbrechend (Brechzahl ≥ 1.8) sein. Als Metalloxide und Metalloxidhydrate eignen sich alle als Schichten aufzubringende Metalloxide oder Metalloxidhydrate, wie z.B. Aluminiumoxid, Aluminiumoxidhydrat, Siliziumdioxid, Siliziumdioxidhydrat, Eisenoxid, Zinnoxid, Ceroxid, Zinkoxid, Zirkoniumoxid, Chromoxid, Titanoxid, insbesondere Titandioxid, Titanoxidhydrat sowie Mischungen hieraus, wie z.B. Ilmenit oder Pseudobrookit. Als Metallsuboxide können beispielsweise die Titansuboxide eingesetzt werden. Als Metalle eignen sich z.B. Chrom, Aluminium, Nickel, Silber, Gold, Titan, Kupfer oder Legierungen, als Metallfluorid eignet sich beispielsweise Magnesiumfluorid. Als Metallnitride oder Metalloxynitride können beispielsweise die Nitride oder Oxynitride der Metalle Titan, Zirkonium und/oder Tantal eingesetzt werden. Bevorzugt sind Metalloxid-, Metall-, Metallfluorid und/oder Metalloxidhydratschichten und ganz besonders bevorzugt Metalloxid-und/oder Metalloxidhydratschichten auf den Substraten aufgebracht. Weiterhin können auch Mehrschichtaufbauten aus hoch- und niedrigbrechenden Metalloxid-, Metalloxidhydrat-, Metall- oder Metallfluoridschichten vorliegen, wobei sich vorzugsweise hoch- und niedrigbrechende Schichten abwechseln. Pigmente umfassend einen mehrschichtigen Aufbau, insbesondere aus alternierend hoch- und niedrigbrechenden Schichten, können einen Farb-Flopp bei Betrachtung aus verschiedenen Betrachtungswinkeln aufweisen. Wenn die erfindungsgemäßen Pigmente in der metallisches Eisen enthaltenden Beschichtung nur geringe oder mittlere Anteile an metallischem Eisen aufweisen, kann auch ein farbiges Aussehen dieser Pigmente und, bei geeignetem Substrat und geeigneter Schichtdicke der metallisches Eisen enthaltenden Beschichtung, auch ein Farb-Flopp bei Betrachtung aus verschiedenen Betrachtungswinkeln erhalten werden, ohne dass außer dem Substrat und der metallisches Eisen enthaltenden Schicht noch weitere Schichten vorhanden sein müssen.

Zusätzlich können bei den erfindungsgemäßen Pigmenten auch auf der metallisches Eisen enthaltenden Schicht noch eine oder mehrere weitere Schichten enthaltend Metalloxide, Metalloxidhydrate, Metallsuboxide, Metallfluoride, Metallnitride, Metalloxynitride und/oder Mischungen hieraus vorliegen. Dabei kommen die bereits vorab beschriebenen Materialien zum Einsatz. Besonders bevorzugt sind Metalloxid- und/oder Metalloxidhydratschichten.
Wenn sich auf der metallisches Eisen enthaltenden Schicht mehr als eine weitere Schicht befindet, ist es wiederum vorteilhaft, den Schichtaufbau so zu gestalten, dass sich hoch- und niedrigbrechende Schichten abwechseln. Dabei ist es bevorzugt, wenn die äußerste Schicht aus einem der genannten hochbrechenden Materialien besteht. Ein solcher Schichtaufbau auf der metallisches Eisen enthaltenden Schicht kann zu deckenden Pigmenten führen, die bunte Interferenzfarben aufweisen. Werden die oben beschriebenen Mehrschichtaufbauten eingesetzt, können außerdem interessante Farbwechselspiele, abhängig vom Beleuchtungs- und/oder Betrachtungswinkel, beobachtet werden.

Besonders geeignete Materialien mit hoher Brechzahl sind beispielsweise TiO₂, ZrO₂, ZnO, SnO₂ und/oder Mischungen hieraus. Besonders bevorzugt ist TiO₂. Die Dicke dieser Schichten beträgt dabei jeweils etwa 3 bis 300 nm und bevorzugt 20 bis 200 nm.

Besonders geeignete Materialien mit niedriger Brechzahl sind beispielsweise SiO₂, SiO(OH)₂, Al₂O₃, AIO(OH), B₂O₃, MgF₂ und/oder Mischungen hieraus. Besonders bevorzugt ist SiO₂. Die Dicke der einzelnen Schichten aus diesen Materialien beträgt zwischen 3 und 300 nm, bevorzugt sind sie dicker als 20 nm und bis zu 200 nm dick.

Grundsätzlich können an der Phasengrenze zwischen der metallisches Eisen enthaltenden Beschichtung und einer zusätzlichen Schicht Reaktionen eintreten, die zur Ausbildung von neuen Phasen, z.B. Legierungen, intermetallischen Verbindungen, neuen oxidischen Zusammensetzungen, führt.

Je nach Material der zusätzlichen Schichten und deren Schichtdicken können die erfindungsgemäßen Pigmente semitransparent oder deckend sein. Dabei gilt grundsätzlich, dass die Pigmente umso deckender sind, je höher der Anteil an metallischem Eisen in der Eisen-haltigen Beschichtung ist.

Darüber hinaus können die erfindungsgemäßen Pigmente auch zusätzliche Eigenschaften aufweisen, die für eine Reihe von Anwendungen, insbesondere für verschiedene Sicherheitsanwendungen, von Interesse sind. So können bei entsprechendem Gehalt an metallischem Eisen in der Eisen-haltigen Beschichtung auch elektrisch leitfähige oder magnetisierbare Pigmente erhalten werden. Dies gilt insbesondere bei Pigmenten mit verhältnismäßig hohen Anteilen von metallischem Eisen in der Beschichtung.

Solche elektrisch leitfähigen und/oder magnetisierbaren Pigmente sind insbesondere für Sicherheitsmerkmale von Interesse, die vorzugsweise auf Wertdokumenten aller Art aufgebracht werden, um deren Fälschung zu verhindern.
Die Kombination von ohne Hilfsmittel sichtbaren Merkmalen (Farbigkeit, Farb-Flopp) mit versteckten Merkmalen (elektrische Leitfähigkeit, Magnetisierbarkeit) ist hier eine häufig gewünschte Eigenschaft von Pigmenten, da Pigmente besonders einfach in Druckfarben eingesetzt werden können und damit die Massenproduktion von Wertdokumenten vereinfachen. Auf diese Weise können Druckbilder erhalten werden, die optisch auffällig und schwer kopierbar sind (Farb-Flopp), die gleichzeitig aber auch über das von ihnen erzeugte magnetisierbare Signal oder eine magnetisierbare Spur, die sich selbst aus einem Druckbild ergibt, welches ein Zufallsmuster aufweist, maschinell ausgewertet werden können.

Auch die elektrische Leitfähigkeit der erfindungsgemäßen Pigmente lässt sich vorteilhaft in Sicherheitsanwendungen ausnutzen, bevorzugt auch hier in Verbindung mit den Farbeigenschaften der Pigmente.

Die Form und Größe der eingesetzten Substrate ist an sich nicht kritisch. Die Substrate können unregelmäßig geformt, sphärisch oder plättchenförmig sein. Sphärische Substrate bestehen beispielsweise aus SiO₂ oder Glas und weisen einen Durchmesser von 0.2 bis 10 *µ*m, vorzugsweise von 0.5 bis 5 *µ*m auf. Vorzugsweise sind die Substrate plättchenförmig. Plättchenförmige Substrate weisen in der Regel eine Dicke zwischen 0.05 und 5 *µ*m, insbesondere zwischen 0.1 und 4.5 *µ*m auf. Dabei haben synthetisch hergestellte Substrate, wie beispielsweise SiO₂ oder Glas, den Vorteil, dass sie in den verschiedenen Dicken für verschiedene koloristische Eigenschaften gezielt hergestellt werden können. Die Ausdehnung der Interferenzpigmente in der Länge bzw. Breite kann zwischen 1 und 250 *µ*m betragen, vorzugsweise liegt sie im Bereich von 2 bis 200 *µ*m und ganz besonders bevorzugt im Bereich von 2 bis 100 µm. Die Größe und Dicke der Substrate kann an die Anforderungen der jeweiligen Applikationen angepasst werden.
Die genannten Substrate sind mit einer Eisen-haltigen Beschichtung umfassend metallisches Eisen versehen, die vorzugsweise als äußere optisch aktive Schicht wirkt. Die Schichtdicke der Eisen-haltigen Beschichtung beträgt 1 bis 300 nm, vorzugsweise 1 bis 100 nm. Der Anteil an metallischen Eisen in der Eisen-haltigen Beschichtung beträgt 30 bis 99 Gew.-%, insbesondere 60 bis 85 Gew.-%, bezogen auf die Eisen-haltige Beschichtung. Das metallische Eisen in der Eisen-haltigen Beschichtung liegt in Kombination mit FeO und/oder Fe₃O₄ vor. Darüber hinaus können in der Eisen-haltigen Beschichtung auch weitere einfache oder komplexe Metalloxide vorliegen, beispielsweise TiO₂, Ilmenit oder Pseudobrookit.
Vorzugsweise ist die Eisen-haltige Beschichtung direkt auf einem plättchenförmigen Substrat, insbesondere aus Glimmer, Glas, SiO₂ oder Al₂O₃, aufgebracht. Liegt beispielsweise eine Eisen-haltige Beschichtung mit einem Anteil von 30 bis 99 Gew.-% an metallischem Eisen auf einem plättchenförmigen SiO₂-Substrat vor, dann werden Pigmente erhalten, die je nach Schichtdicke des Substrats und der Eisen-haltigen Beschichtung verschiedene Farb-Flopps aufweisen (beispielsweise rot-gold oder goldgrün), die ein hohes Deckvermögen aufweisen und gleichzeitig elektrisch leitfähig und/oder magnetisierbar sind.
In einer weiteren Ausführungsform können die erfindungsgemäßen Pigmente mit einer zusätzlichen anorganischen und/oder organischen Beschichtung versehen sein. Beispiele für derartige Beschichtungen finden sich z.B. in EP 0 632 109, US 5,759,255, DE 43 17 019, DE 39 29 423, DE 32 35 017, EP 0 492 223, EP 0 342 533, EP 0 268 918, EP 0 141 174, EP 0 764 191, WO 98/13426 oder EP 0 465 805.
Pigmente enthaltend eine organische Beschichtung, z.B. aus Organosilanen oder Organotitanaten bzw. Organozirkonaten zeigen neben den bereits genannten optischen Eigenschaften zusätzlich eine erhöhte Stabilität gegenüber Witterungseinflüssen, wie z.B. Feuchtigkeit und Licht, was vor allem für Industrielacke und im Automobilbereich von besonderem Interesse ist. Die Stabilisierung kann durch anorganische Komponenten der zusätzlichen Beschichtung verbessert werden. Darüber hinaus ist es auch möglich, dass die zusätzliche Beschichtung auf anorganischen Materialien basiert, insbesondere auf Oxiden und Oxidhydraten der Elemente Silizium, Aluminium, Zink, Zinn, Cer und/oder Zirkon. Insgesamt sind die jeweiligen Anteile für die zusätzliche stabilisierende Beschichtung so auszuwählen, dass die optischen Eigenschaften der erfindungsgemäßen Pigmente nicht wesentlich beeinflusst werden.

Die erfindungsgemäßen Pigmente sind erhältlich durch Umsetzung eines gegebenenfalls zusätzlich mit einer oder mehreren Schichten beschichteten Substrats, welches als äußerste Schicht eine Eisenoxid-haltige Beschichtung aufweist, in einem reduzierenden Gasgemisch aus Stickstoff und Wasserstoff unter Bildung von metallischem Eisen, sowie gegebenenfalls weiteres Beschichten des so erhaltenen Pigmente mit einer oder mehreren zusätzlichen Schichten. Dies ermöglicht die Bereitstellung der Eisen-haltigen Beschichtung enthaltend metallisches Eisen, wobei sich die metallisches Eisen enthaltende Schicht direkt auf dem Substrat, zwischen anderen Schichten oder als äußerste Schicht auf dem Substrat befinden kann.

Allgemein sind Reduktionen von Metalloxidschichten bei Glanzpigmenten bekannt, z.B. aus DE 199 53 655, DE 198 43 014, DE 198 22 046, DE 196 18 562, DE 195 11 697 oder DE 195 11 696. Die in den genannten Dokumenten beschriebenen Reduktionsverfahren unterscheiden sich aber deutlich in der Vorgehensweise von derjenigen gemäß der vorliegenden Erfindung. Vielfach werden andere Reduktionsmittel, z.B. Ammoniak, Kohlenstoff, Kohlenwasserstoffe oder Metalle, eingesetzt. Wesentlicher Nachteil des Einsatzes der genannten Reduktionsmittel ist die Verunreinigung der damit reduzierten Schicht, insbesondere mit Kohlenstoff, die zu unerwünschten Veränderungen der eigentlich gewünschten Farbeffekte führt. Auch die Reduktion mit Metallen ist nachteilig, weil auf diese Weise eine zusätzliche Komponente in die Beschichtung mit eingebracht wird, die ebenfalls zu unerwünschten Veränderungen der Eigenschaften der Pigmente führen kann.

Darüber hinaus findet bei allen Pigmenten aus dem Stand der Technik eine Reduktion des als oberste Schicht vorliegenden Titandioxids statt, die zur Bildung bläulich gefärbter Titansuboxide führt. Diese gefärbten Suboxide sind das maßgebliche Ziel der Reduktion. Eine Reduktion Eisenoxidhaltiger Beschichtungen bis zum metallischen Eisen ist nicht bekannt. Es war auch nicht zu erwarten, dass die erfindungsgemäßen Pigmente auf diese Weise erhältlich sind, weil sich üblicherweise bei der Reduktion zunächst Eisenoxide wie Fe₃O₄ oder FeO bilden.

Im erfindungsgemäßen Verfahren zur Herstellung der beschriebenen Pigmente wird ein mit einer Eisenoxid-haltigen Beschichtung beschichtetes Substrat, welches gegebenenfalls zwischen dem Substrat und der Eisenoxid-haltigen Schicht eine oder mehrere weitere Schichten aufweisen kann, in einem reduzierenden Gasgemisch aus Stickstoff und Wasserstoff unter Bildung von metallischem Eisen umgesetzt. Auf diese Weise wird sichergestellt, dass die metallisches Eisen enthaltende Beschichtung frei von Kohlenstoff ist. Das in der Eisenoxid-haltigen Beschichtung enthaltene Eisenoxid ist vorzugsweise Eisen(III)-oxid. Eisen(III)-oxid-haltige Beschichtungen lassen sich in für den Fachmann bekannter Weise herstellen, z.B. nasschemisch durch Ausfällung aus entsprechenden Eisensalzen in wässriger Lösung oder einem Wasser/Lösemittel-Gemisch. Fällungen dieser Art sind z.B. aus DE 2313331 bekannt.

Das für die Umsetzung einzusetzende Gasgemisch aus Stickstoff und Wasserstoff hat einen Anteil von Wasserstoff im Bereich von 2.5 bis 25 Vol.-%, insbesondere von 4 bis 10 Vol.-%, und ganz besonders bevorzugt von 5 bis 8 Vol.-%.

Die Reduktion der Eisenoxid-haltigen Beschichtung erfolgt bei Temperaturen von 400 bis 1000°C, vorzugsweise von 500 bis 900°C und insbesondere bevorzugt von 550 bis 850°C. Die Glühdauer beträgt 15-240 Minuten, vorzugsweise 30-120 Minuten und insbesondere 30-90 Minuten

Anschließend kann das so erhaltene Pigment bei Bedarf noch mit einer oder mehreren weiteren Schichten beschichtet werden, deren Zusammensetzung bereits vorab beschrieben wurde. Diese Schichten können, wie die gegebenenfalls ebenfalls vorhandenen Schichten auf dem Substrat unterhalb der Eisenoxid-haltigen Schicht auch, nach bekannten Verfahren aufgebracht werden, beispielsweise nasschemisch durch Ausfällung aus entsprechenden Metallsalzen in wässriger Lösung, durch Abscheidung aus organischen Metallverbindungen im Wirbelbett sowie durch CVD oder PVD-Verfahren. Solche Verfahren werden üblicherweise zur Beschichtung von Pigmentsubstraten eingesetzt und sind beispielsweise in DE 14 67 468, DE 19 59 988, DE 20 09 566, DE 22 14 545, DE 22 15 191, DE 22 44 298, DE 23 13 331, DE 25 22 572, DE 31 37 808, DE 31 37 809,DE 31 51 343, DE 31 51 354, DE 31 51 355, DE 32 11 602, DE 32 35 017 beschrieben.

Darüber hinaus kann in einem ebenfalls erfindungsgemäßen Verfahren zusätzlich als äußere Schicht eine anorganische und/oder organische Beschichtung aufgebracht werden. Beispiele für derartige Beschichtungsverfahren finden sich unter anderem in EP 0 632 109, US 5,759,255, DE 43 17 019, DE 39 29 423, DE 32 35 017, EP 0 492 223, EP 0 342 533, EP 0 268 918, EP 0 141 174, EP 0 764 191, WO 98/13426 oder EP 0 465 805. Beispiele für organische Beschichtungen sowie die damit verbundenen Vorteile sind bereits vorab beim Aufbau der erfindungsgemäßen Pigmente beschrieben worden. Der Verfahrensschritt der Aufbringung der organischen Beschichtung kann direkt an die anderen Schritte des erfindungsgemäßen Verfahrens angeschlossen werden. Die hierbei aufgebrachten Stoffe umfassen lediglich einen Gewichtsanteil von 0.1 bis 5 Gew. %, vorzugsweise 0.5 bis 3 Gew. %, des gesamten Pigmentes.

Die erfindungsgemäßen Pigmente sind vielseitig einsetzbar und können in vielen Bereichen eingesetzt werden. Demgemäss ist die Verwendung der erfindungsgemäßen Pigmente in Kosmetika, Lacken, Farben, Kunststoffen, Folien, in Sicherheitsanwendungen, zur Saatguteinfärbung, zur Lebensmitteleinfärbung oder in Arzneimittelüberzügen, zur Lasermarkierung sowie zur Herstellung von Pigmentpräparationen und Trockenpräparaten ebenfalls Gegenstand der vorliegenden Erfindung.

Im Falle von Kosmetika eignen sich die erfindungsgemäßen Pigmente besonders für Produkte und Formulierungen der dekorativen Kosmetik, wie z. B. Nagellacke, farbgebende Puder, Lippenstifte oder Lidschatten, Seifen, Zahnpasten etc. Selbstverständlich können die erfindungsgemäßen Interferenzpigmente in den Formulierungen auch mit jeder Art von kosmetischen Roh- und Hilfsstoffen kombiniert werden. Dazu gehören u. a. Öle, Fette, Wachse, Filmbildner, Konservierungsmittel und allgemein anwendungstechnische Eigenschaften bestimmende Hilfsstoffe, wie z. B. Verdicker und rheologische Zusatzstoffe wie etwa Bentonite, Hektorite, Siliziumdioxid, Ca-Silikate, Gelatine, hochmolekulare Kohlenhydrate und/oder oberflächenaktive Hilfsmittel, etc. Die erfindungsgemäßen Interferenzpigmente enthaltenden Formulierungen können dem lipophilen, hydrophilen oder hydrophoben Typ angehören. Bei heterogenen Formulierungen mit diskreten wässrigen und nicht-wässrigen Phasen können die erfindungsgemäßen Partikel in jeweils nur einer der beiden Phasen enthalten oder auch über beide Phasen verteilt sein.

Die pH-Werte der wässrigen Formulierungen können zwischen 1 und 14, bevorzugt zwischen 2 und 11 und besonders bevorzugt zwischen 5 und 8 liegen. Den Konzentrationen der erfindungsgemäßen Interferenzpigmente in der Formulierung sind keine Grenzen gesetzt. Sie können - je nach Anwendungsfall - zwischen 0.001 (rinse-off-Produkte, z. B. Duschgele) und 99 % (z. B. Glanzeffekt-Artikel für besondere Anwendungen) liegen. Die erfindungsgemäßen Interferenzpigmente können weiterhin auch mit kosmetischen Wirkstoffen kombiniert werden. Geeignete Wirkstoffe sind z. B. Insect Repellents, UV A/BC-Schutzfilter (z. B. OMC, B3, MBC), Anti-Ageing-Wirkstoffe, Vitamine und deren Derivate (z. B. Vitamin A, C, E etc.), Selbstbräuner (z. B. DHA, Erythrolose u.a.) sowie weitere kosmetische Wirkstoffe wie z. B. Bisabolol, LPO, Ectoin, Emblica, Allantoin, Bioflavanoide und deren Derivate.

Bei Einsatz der Pigmente in Lacken und Farben sind alle dem Fachmann bekannten Anwendungsbereiche möglich, wie z. B. Pulverlacke, Automobillacke, Druckfarben für den Tief-, Offset-, Sieb- oder Flexodruck sowie für Lacke in Außenanwendungen. Vorzugsweise basieren die für den Druck eingesetzten erfindungsgemäßen Pigmente auf plättchenförmigen Substraten. Die Lacke und Farben können hierbei beispielsweise strahlungshärtend, physikalisch trocknend oder chemisch härtend sein. Für die Herstellung der Druckfarben oder Flüssiglacke ist eine Vielzahl von Bindern, z.B. auf der Basis von Acrylaten, Methacrylaten, Polyestern, Polyurethanen, Nitrocellulose, Ethylcellulose, Polyamid, Polyvinylbutyrat, Phenolharzen, Maleinharzen, Stärke oder Polyvinylalkohol, Aminharzen, Alkydharzen, Epoxidharzen, Polytetrafluorethylen, Polyvinylidenfluoriden, Polyvinylchlorid oder Mischungen hieraus geeignet, insbesondere wasserlösliche Typen. Bei den Lacken kann es sich um Pulverlacke oder wasser- oder lösemittelbasierte Lacke handeln, wobei die Auswahl der Lackbestandteile dem Allgemeinwissen des Fachmanns unterliegt. Gängige polymere Bindemittel für Pulverlacke sind beispielsweise Polyester, Epoxide, Polyurethane, Acrylate oder Mischungen hieraus.

Darüber hinaus können die erfindungsgemäßen Pigmente in Folien und Kunststoffen verwendet werden, so z. B. in Agrarfolien, infrarotreflektierenden Folien und Scheiben, Geschenkfolien, Kunststoffbehältnissen und Formkörpern für alle dem Fachmann bekannten Anwendungen. Als Kunststoffe eignen sich alle gängigen Kunststoffe für die Einarbeitung der erfindungsgemäßen Interferenzpigmente, z. B. Duromere oder thermoplastische Kunststoffe. Die Beschreibung der Anwendungsmöglichkeiten und der einsetzbaren Kunststoffe, Verarbeitungsverfahren und Additive finden sich z. B. in der RD 472005 oder in R. Glausch, M. Kieser, R. Maisch, G. Pfaff, J. Weitzel, Perlglanzpigmente, Curt R. Vincentz Verlag, 1996, 83 ff., deren Offenbarungsgehalt hier mit umfasst ist.

Wie bereits vorab beschrieben, lassen sich die erfindungsgemäßen Pigmente besonders vorteilhaft in Sicherheitsanwendungen einsetzen. Unter Sicherheitsanwendung wird dabei jede Anwendung verstanden, bei der Produkte mit Sicherheitsmerkmalen versehen werden, die die Echtheit der Produkte nachweisbar macht und/oder zum Schutz vor Fälschungen, zum Gewährleisten einer Zutrittsberechtigung etc. eingesetzt wird, beziehungsweise die Anwendung in Geräten und Messeinrichtungen zur Überprüfung dieser Eigenschaften.
Vorzugsweise kann der Einsatz der erfindungsgemäßen Pigmente in Sicherheitsmerkmalen von Wertdokumenten erfolgen. Unter Wertdokumenten sind Produkte wie Banknoten, Schecks, Kreditkarten, Aktien, Pässe, Ausweisdokumente, Zutrittsberechtigungsausweise, Führerscheine, Eintrittskarten, Wertmarken, Briefmarken, Etiketten, Siegel und dergleichen zu verstehen. Die erfindungsgemäßen Pigmente sind jedoch auch in Sicherheitsmerkmalen in Verpackungsmaterialien wie beispielsweise Verpackungen von Medikamenten, Lebensmitteln, Parfüms Zigaretten und ähnlichem, oder direkt auf Produkten des alltäglichen Gebrauchs, wie beispielsweise Bekleidung, Schuhen, Haushaltsartikeln, Haushaltselektronikartikeln und ähnlichem, einsetzbar. Dabei ist jede Form eines Sicherheitsmerkmals, die Pigmente enthalten kann, geeignet, beispielsweise Etiketten, ganz- oder teilflächige Beschichtungen, Drucke, holographische Elemente etc. Das Sicherheitsmerkmal kann dabei einoder mehrschichtig aufgebaut sein und mit einem oder mehreren anderen Sicherheitsmerkmalen derselben oder anderer Art kombiniert vorliegen.

Vorzugsweise werden die erfindungsgemäßen Pigmente in Druckfarben für den Sicherheitsdruck eingesetzt, da sie sich in den üblichen Druckfarben gut dispergieren lassen und Druckverfahren zur Herstellung von Sicherheitsmerkmalen sehr variabel eingesetzt werden können.

Die erfindungsgemäßen Pigmente lassen sich jedoch auch mit gutem Erfolg in die Basismaterialien für Sicherheitsprodukte, beispielsweise in Papier, papierähnliche Stoffe wie Kartons, Pappen und dergleichen und/oder Kunststoffe direkt einarbeiten. Dabei wird eine statistische Verteilung der Pigmente erzielt, die an Hand ihrer optischen und/oder funktionellen Eigenschaften im Endprodukt nachweisbar sind.

Wie bereits vorab beschrieben, ist in Sicherheitsanwendungen die Kombination von sichtbaren Eigenschaften mit funktionellen Eigenschaften (Magnetisierbarkeit, elektrische Leitfähigkeit), die bei den erfindungsgemäßen Pigmenten vorliegen kann, besonders vorteilhaft einsetzbar. Bevorzugt werden deshalb für Sicherheitsanwendungen solche erfindungsgemäßen Pigmente eingesetzt, die ein plättchenförmiges Substrat aufweisen und farbig sind, und insbesondere solche, die einen Farb-Flopp zeigen, wenn sie in Beschichtungen aus unterschiedlichen Winkeln betrachtet werden. Allein diese Eigenschaft verleiht Druckbildern, Beschichtungen etc., die die erfindungsgemäßen Pigmente enthalten, eine Farbgestaltung, die gut erkennbar und nicht mit Kopiergeräten kopierbar ist.

Darüber hinaus können die funktionellen Eigenschaften der Pigmente dazu benutzt werden, entweder diese Eigenschaft (z. B. elektrische Leitfähigkeit, Magnetisierbarkeit) als solche qualitativ und/oder quantitativ zu detektieren, oder aber über Beschichtungen in Form von Mustern, Linien, Logos, alphanumerischen Zeichen etc., diese durch ganze oder teilweise Ausführung mittels der erfindungsgemäßen Pigmente auch maschinenlesbar darzustellen. So kann die Echtheit von Dokumenten zum Beispiel dadurch nachgewiesen werden, dass ein Druckbild, welches vorteilhafterweise optisch variabel ist, das heißt einen Farb-Flopp aufweist, wenn es unter natürlicher oder künstlicher Lichteinwirkung betrachtet wird, einen nur maschinell sichtbaren und/oder detektierbaren elektrisch leitfähigen oder magnetisierbaren "Fingerabdruck" enthält, der dem Dokument eine weitere Sicherheitsstufe verleiht. Dabei reicht es aus, wenn der "Fingerabdruck" lediglich das Druckbild der einzelnen Pigmente auf einer vorbestimmten Fläche beinhaltet, wie es beim Drucken zufällig entsteht. Solche Zufallsbilder können von Computern technisch bei der Herstellung der Produkte erfasst und bei der späteren Echtheitsprüfung dieser Produkte wieder abgerufen werden. Beschichtungen, die die erfindungsgemäßen Pigmente enthalten, lassen sich außerdem im noch feuchten Zustand mit üblichen Festmagneten beschreiben bzw. codieren, wodurch sowohl sichtbare als auch maschinenlesbare Sicherheitsmerkmale erzeugt werden können.

Von besonderem Vorteil ist es, dass die erfindungsgemäßen Pigmente bei geeignetem Substrat und geeigneten Schichtdicken sowohl die wünschenswerten optischen (Farb-Flopp) als auch die wünschenswerten funktionellen Eigenschaften in sich vereinen. Hierfür sind besonders die erfindungsgemäßen Pigmente geeignet, die ein weitgehend transparentes, plättchenförmiges Substrat, beispielsweise aus Glimmer, Glas oder SiO₂, aufweisen.

Im Bereich der Landwirtschaft können die Pigmente zur Einfärbung von Saatgut und anderen Ausgangsgütern verwendet werden, darüber hinaus im Lebensmittelbereich zur Pigmentierung von Lebensmitteln. Zur Pigmentierung von Überzügen in Arzneimitteln wie z. B. Tabletten oder Dragees sind die erfindungsgemäßen Pigmente ebenfalls einsetzbar.

Für die Lasermarkierung unter Verwendung der erfindungsgemäßen Pigmente können alle bekannten thermoplastischen Kunststoffe, wie sie z.B. im Ullmann, Bd. 15, S. 457 ff., Verlag VCH beschrieben werden, sowie Papiere aller bekannten Arten und Zusammensetzungen verwendet werden. Geeignete Kunststoffe sind z.B. Polyethylen, Polypropylen, Polyamide, Polyester, Polyesterester, Polyetherester, Polyphenylenether, Polyacetal, Polybutylenterephthalat, Polymethylacrylat, Polyvinylacetat, Polystyrol, Acrylnitril-Butadien-Styrol, Acrylnitril-Styrol-Acrylester, Polycarbonat, Polyethersulfone, Polyetherketone sowie deren Copolymere und/oder Mischungen.

Die Einarbeitung der erfindungsgemäßen Pigmente in den thermoplastischen Kunststoff erfolgt, indem das Kunststoffgranulat mit dem Interferenzpigment gemischt und dann unter Wärmeeinwirkung verformt wird. Dem Kunststoffgranulat können bei der Einarbeitung der Interferenzpigmente dem Fachmann bekannte Haftmittel, organische polymerverträgliche Lösemittel, Stabilisatoren und/oder unter den Arbeitsbedingungen temperaturstabile Tenside zugesetzt werden. Die Herstellung der pigmentierten Kunststoffgranulate erfolgt in der Regel so, dass in einem geeigneten Mischer das Kunststoffgranulat vorgelegt, mit eventuellen Zusätzen benetzt und danach das Interferenzpigment zugesetzt und untergemischt wird. Die so erhaltene Mischung kann dann direkt in einem Extruder oder einer Spritzgussmaschine verarbeitet werden. Anschließend findet die Markierung mit geeigneter Strahlung statt.

Im Falle von Papier kann die Einarbeitung der Pigmente in die Beschichtung und/oder in die Papiermasse erfolgen.

Vorzugsweise wird bei der Markierung energiereiche Strahlung eingesetzt, im allgemeinen im Wellenlängenbereich von 157 bis 10600 nm, insbesondere im Bereich von 300 bis 10600 nm. Beispielsweise seien hier CO₂-Laser (10600 nm), Nd:YAG-Laser (1064 bzw. 532 nm) oder gepulste UV-Laser (Excimer-Laser) erwähnt. Die Excimerlaser weisen folgende Wellenlängen auf: F₂-Excimerlaser (157 nm), ArF-Excimerlaser (193 nm), KrCI-Excimerlaser (222 nm), KrF-Excimerlaser (248 nm), XeCI-Excimerlaser (308 nm), XeF-Excimerlaser (351 nm), frequenzvervielfachte Nd : YAG-Laser mit Wellenlängen von 355 nm (frequenzverdreifacht) oder 265 nm (frequenzvervierfacht). Besonders bevorzugt werden Nd : YAG-Laser (1064 bzw. 532 nm) und CO₂-Laser eingesetzt. Die Energiedichten der eingesetzten Laser liegen im allgemeinen im Bereich von 0,3 mJ/cm² bis 50 J/cm², vorzugsweise 0,3 mJ/cm² bis 10 J/cm².

Die Beschriftung mit dem Laser erfolgt derart, dass der Probenkörper in den Strahlengang eines gepulsten Lasers, vorzugsweise eines CO₂- oder Nd : YAG-Lasers gebracht wird. Ferner ist eine Beschriftung mit einem Excimer-Laser, z. B. über eine Maskentechnik, möglich. Jedoch sind auch mit anderen herkömmlichen Lasertypen, die eine Wellenlänge in einem Bereich hoher Absorption der verwendeten laserlichtabsorbierenden Substanz aufweisen, die gewünschten Ergebnisse zu erzielen. Die erhaltene Markierung wird durch die Bestrahlungszeit (bzw. Pulszahl bei Pulslasern) und Bestrahlungsleistung des Lasers sowie des verwendeten Kunststoffsystems bzw. Lacksystems bestimmt. Die Leistung der verwendeten Laser hängt von der jeweiligen Anwendung ab und kann im Einzelfall vom Fachmann ohne weiteres ermittelt werden.

Bei der Verwendung von gepulsten Lasern liegt die Pulsfrequenz im allgemeinen im Bereich von 1 bis 30 kHz. Entsprechende Laser, die im erfindungsgemäßen Verfahren eingesetzt werden können, sind kommerziell erhältlich.

Die Verwendung der erfindungsgemäßen Pigmente zur Lasermarkierung kann in allen oben genannten Kunststoffen und im Papier erfolgen. Die auf diese Weise pigmentierten Kunststoffe können als Formkörper in der Elektro-, Elektronik- und Kraftfahrzeugindustrie Anwendung finden. Ein weiteres wichtiges Anwendungsgebiet für die Laserbeschriftung sind Wertdokumente verschiedenster Art und Kunststoffmarken zur individuellen Kennzeichnung von Tieren. Der Anteil an Pigmente im Kunststoff beträgt im Falle der Lasermarkierung bei den Anwendungen 0.01 bis 10 Gew.-%, vorzugsweise 0.05 bis 5 Gew.-% und insbesondere 0.1 bis 3 Gew.-%. Die Kennzeichnung und Beschriftung von Gehäusen, Leitungen, Tastenkappen, Zierleisten bzw. Funktionsteilen im Heizungs-, Lüftungs-und Kühlbereich oder Schalter, Stecker, Hebel und Griffe, die aus den mit den erfindungsgemäßen Pigmenten pigmentierten Kunststoffen bestehen, kann selbst an schwer zugänglichen Stellen mit Hilfe von Laserlicht erfolgen. Die Markierungen zeichnen sich dadurch aus, dass sie wisch- und kratzfest, stabil bei nachträglichen Sterilisationsprozessen und hygienisch rein beim Markierungsprozess aufbringbar sind.

Die erfindungsgemäßen Pigmente eignen sich in den oben genannten Anwendungsgebieten ebenso zur Verwendung in Abmischungen mit allen bekannten organischen oder anorganischen Farbstoffen und/oder Pigmenten. Organische Pigmente und Farbstoffe sind beispielsweise Monoazopigmente, Disazopigmente, polycyclische Pigmente, kationische, anionische oder nichtionische Farbstoffe. Anorganische Farbstoffe und Pigmente sind beispielsweise Weißpigmente, Buntpigmente, Schwarzpigmente oder Effektpigmente. Beispiele für geeignete Effektpigmente sind Metalleffektpigmente, Perlglanzpigmente oder Interferenzpigmente, die in der Regel auf ein- oder mehrfach beschichteten Plättchen auf Basis von Glimmer, Glas, Al₂O₃, Fe₂O₃, SiO₂, etc. beruhen.

Beispiele für Aufbauten und besondere Eigenschaften der genannten Pigmente finden sich beispielsweise in RD 471001 oder RD 472005, deren Offenbarung hiermit unter Bezugnahme in der vorliegenden Erfindung mit eingeschlossen ist. Darüber hinaus eignen sich als weitere Farbmittel zur Abmischung mit den erfindungsgemäßen Pigmenten lumineszierende Farbstoffe und/oder Pigmente sowie holographische Pigmente oder LCPs (Liquid Crystal Polymers). Die erfindungsgemäßen Pigmente können in jedem Verhältnis mit handelsüblichen Pigmenten und Füllern gemischt werden.

Wenn die Pigmente eine dunkle Körperfarbe aufweisen, können sie auch in unterschiedlichen Formulierungen an Stelle von Ruß zur Erzeugung von Deckvermögen eingesetzt werden.

Als Füllstoffe sind z. B. natürlicher und synthetischer Glimmer, Nylon Powder, reine oder gefüllte Melaminharze, Talcum, Gläser, Kaolin, Oxide oder Hydroxide von Aluminium, Magnesium, Calcium, Zink, BiOCl, Bariumsulfat, Calciumsulfat, Calciumcarbonat, Magnesiumcarbonat, Kohlenstoff, sowie physikalische oder chemische Kombinationen dieser Stoffe zu nennen. Bezüglich der Partikelform des Füllstoffes gibt es keine Einschränkungen. Sie kann den Anforderungen gemäß z. B. plättchenförmig, sphärisch oder nadelförmig sein.

Die erfindungsgemäßen Pigmente sind weiterhin geeignet zur Herstellung von fließfähigen Pigmentpräparationen und Trockenpräparaten enthaltend ein oder mehrere erfindungsgemäße Partikel, Bindemittel und optional ein oder mehrere Additive. Unter Trockenpräparate sind auch Präparate zu verstehen, die 0 bis 8 Gew.-%, vorzugsweise 2 bis 8 Gew.-%, insbesondere 3 bis 6 Gew.-%, an Wasser und/oder eines Lösemittels oder Lösemittelgemisches enthalten. Die Trockenpräparate liegen vorzugsweise als Pellets, Granulate, Chips, Würstchen oder Briketts vor und weisen Teilchengrößen von 0.2 - 80 mm auf. Die Trockenpräparate finden insbesondere Anwendung bei der Herstellung von Druckfarben und in kosmetischen Formulierungen.

Die nachfolgenden Beispiele sollen die Erfindung näher erläutern, ohne sie jedoch zu begrenzen.

### Beispiele

### Beispiel 1:

100 g Glimmer der Teilchengröße 10-60 µm werden in 1.9 I entmineralisiertem Wasser unter Rühren auf 75°C erhitzt. Der pH-Wert der Suspension wird mit 10 %-iger Salzsäure auf 3.0 eingestellt. Nun werden 200 g einer 30 %-igen FeCl₃-Lösung zudosiert, wobei der pH-Wert durch gleichzeitiges Zutropfen einer 32 %-igen Natronlauge konstant gehalten wird. Das Produkt wird abfiltriert, gewaschen, getrocknet und bei 750°C in einem Gasgemisch aus Stickstoff und Wasserstoff (Anteil an Wasserstoff: 8 Vol.-%) reduziert.

Man erhält ein glänzendes Pigment mit silberner Interferenz, grauschwarzer Körperfarbe und hohem Glanz, dessen Beschichtung aus 74 Gew.-% metallischen Eisens besteht.

### Beispiel 2:

Das getrocknete Pigment aus Beispiel 2 wird bei 800°C in einem Gasgemisch aus Stickstoff und Wasserstoff (Anteil an Wasserstoff: 8 Vol.-%) reduziert. Man erhält ein glänzendes Pigment mit goldfarbener Interferenz, grauschwarzer Körperfarbe und hohem Glanz, dessen Beschichtung aus 80 Gew.-% metallischen Eisens besteht.

### Beispiel 3:

100 g SiO₂-flakes (Dicke 365 nm) der Teilchengröße 10-60 µm werden in 1.9 I entmineralisiertem Wasser unter Rühren auf 85°C erhitzt. Der pH-Wert der Suspension wird mit 10 %-iger Salzsäure auf 2.8 eingestellt. Nun werden 1149 g einer 7 %-igen FeCl₃-Lösung zudosiert, wobei der pH-Wert durch gleichzeitiges Zutropfen einer 32 %-igen Natronlauge konstant gehalten wird. Das Produkt wird abfiltriert, gewaschen, getrocknet und bei 700°C in einem Gasgemisch aus Stickstoff und Wasserstoff (Anteil an Wasserstoff: 8 Vol.-%) reduziert. Man erhält ein glänzendes, deckendes Pigment mit rot nach gold verlaufendem Farbflop und hohem Glanz, dessen Beschichtung aus 44.5 Gew.-% metallischen Eisens besteht.

### Vergleichsbeispiel (entsprechend EP 0246523):

100 g Glimmer der Teilchengröße 10-60 µm werden in 2.5 l entmineralisiertem Wasser unter Rühren auf 80°C erhitzt. Der pH-Wert der Suspension wird mit 15% iger Natronlauge auf 8.0 eingestellt. Nun wird eine Lösung bestehend aus 600 g FeSO₄*7 H₂O, 50 ml konzentrierter Schwefelsäure und 2000 ml Wasser und gleichzeitig eine zweite Lösung, bestehend aus 150 g KNO₃ und 2000 ml Wasser, innerhalb einer Stunde zudosiert, wobei der pH-Wert durch Zugabe von 15% iger Natronlauge konstant gehalten wird. Das Produkt wird abfiltriert, gewaschen und 3 Stunden bei 100°C getrocknet.

Man erhält ein nicht glänzendes, mattes, schwarzes Pigment, dessen Beschichtung aus 100 Gew.-% Magnetit besteht.

### Lasermarkierung:

Ein PP-Granulat (PP-HD, Stamylan PPH 10 der Fa. DSM) wird durch Zusatz von 0.1 Gew.-% des Pigmentes aus Beispiel 1 im Spritzguss verarbeitet. Das erhaltene Formteil (Plättchen) wird anschließend mit einem SHT-Nd:YAG-Laser beschriftet. Bei einer Pulsfrequenz von 2.5 kHz und einer Schreibgeschwindigkeit von 300 mm/s zeigen die Plättchen eine schwarze, kontrastreiche und abriebfeste Beschriftung. Mit steigender Energiedichte wird die Beschriftung zunehmend dunkler.

## Patentansprüche

1. Pigmente umfassend ein Substrat und eine metallisches Eisen enthaltende Beschichtung, wobei das Substrat Titanoxide, synthetischen oder natürlichen Glimmer, Schichtsilikate, Glas, Borosilikate, SiO₂, Al₂O₃, Graphit, und/oder BiOCl umfasst, das metallische Eisen in der Eisen-haltigen Beschichtung in Kombination mit FeO und/oder Fe₃O₄ vorliegt und der Anteil an metallischem Eisen 30 bis 99 Gew.-% bezogen auf die Eisen-haltige Beschichtung beträgt.

2. Pigmente gemäß Anspruch 1, **dadurch gekennzeichnet, dass** der Anteil an metallischem Eisen in der Eisen-haltigen Beschichtung 60 bis 85 Gew.-% bezogen auf die Eisen-haltige Beschichtung beträgt.

3. Pigmente gemäß einem oder mehreren der Ansprüche 1 bis 2, erhältlich durch Umsetzung eines gegebenenfalls zusätzlich mit einer oder mehreren Schichten beschichteten Substrats, welches als äußerste Schicht eine Eisenoxid-haltige Beschichtung aufweist, in einem reduzierenden Gasgemisch aus Stickstoff und Wasserstoff unter Bildung von metallischem Eisen, sowie gegebenenfalls weiteres Beschichten mit ein oder mehreren weiteren Schichten.

4. Pigmente gemäß einem oder mehreren der Ansprüche 1 bis 3, **dadurch gekennzeichnet, dass** das Substrat plättchenförmig ist.

5. Pigmente gemäß einem oder mehreren der Ansprüche 1 bis 4, **dadurch gekennzeichnet, dass** zwischen der metallisches Eisen enthaltenden Beschichtung und dem Substrat zusätzlich eine oder mehrere Schichten enthaltend Metalloxide, Metalloxidhydrate, Metallsuboxide, Metalle, Metallfluoride, Metallnitride, Metalloxynitride und/oder Mischungen hieraus vorliegen.

6. Pigmente gemäß einem oder mehreren der Ansprüche 1 bis 5, **dadurch gekennzeichnet, dass** auf der metallisches Eisen enthaltenden Beschichtung zusätzlich ein oder mehrere Schichten enthaltend Metalloxide, Metalloxidhydrate, Metallsuboxide, Metallfluoride, Metallnitride, Metalloxynitride und/oder Mischungen hieraus vorhanden sind.

7. Pigmente gemäß einem oder mehreren der Ansprüche 1 bis 6, **dadurch gekennzeichnet, dass** sie weiterhin mit einer zusätzlichen anorganischen und/oder organischen Beschichtung als äußere Schicht versehen sind.

8. Verfahren zur Herstellung von Pigmenten gemäß einem oder mehreren der Ansprüche 1 bis 7, wobei ein gegebenenfalls zusätzlich mit einer oder mehreren Schichten beschichtetes Substrat, welches als äußerste Schicht eine Eisenoxid-haltige Beschichtung aufweist, in einem reduzierenden Gasgemisch aus Stickstoff und Wasserstoff unter Bildung von metallischem Eisen umgesetzt sowie gegebenenfalls mit einer oder mehreren weiteren Schichten beschichtet wird.

9. Verfahren gemäß Anspruch 8, **dadurch gekennzeichnet, dass** der Anteil an Wasserstoff in dem Gasgemisch aus Stickstoff und Wasserstoff 2.5 bis 25 Vol.-% beträgt.

10. Verfahren gemäß Anspruch 8 oder 9, **dadurch gekennzeichnet, dass** die Reduktion bei Temperaturen von 400 bis 1000°C erfolgt.

11. Verfahren gemäß einem oder mehreren der Ansprüche 8 bis 10, **dadurch gekennzeichnet, dass** zusätzlich als äußere Schicht eine anorganische und/oder organische Beschichtung aufgebracht wird.

12. Verwendung von Pigmenten gemäß einem oder mehreren der Ansprüche 1 bis 7 in Kosmetika, Lacken, Farben, Kunststoffen, Folien, in Sicherheitsanwendungen, zur Saatguteinfärbung, zur Lebensmitteleinfärbung, zur Lasermarkierung oder in Arzneimittelüberzügen sowie zur Herstellung von Pigmentpräparationen und Trockenpräparaten.

13. Verwendung gemäß Anspruch 12, **dadurch gekennzeichnet, dass** die Pigmente gemäß einem oder mehreren der Ansprüche 1 bis 7 in Abmischung mit organischen oder anorganischen Farbstoffen und/oder Pigmenten vorliegen.

14. Sicherheitsanwendung, **dadurch gekennzeichnet, dass** Wertdokumente, insbesondere Banknoten, Schecks, Kreditkarten, Aktien, Pässe, Ausweisdokumente, Zutrittsberechtigungsausweise, Führerscheine, Eintrittskarten, Wertmarken, Briefmarken, Etiketten, Siegel, Verpackungsmaterialien, insbesondere Verpackungen von Medikamenten, Lebensmitteln, Parfüms, Zigaretten, oder Produkte des alltäglichen Gebrauchs, insbesondere Bekleidung, Schuhe, Haushaltsartikel, Haushaltselektronikartikel, mit mindestens einem Sicherheitsmerkmal versehen werden, welches mindestens ein Pigment gemäß einem oder mehreren der Ansprüche 1 bis 7 enthält.

15. Sicherheitsanwendung gemäß Anspruch 14, **dadurch gekennzeichnet, dass** das Sicherheitsmerkmal ein gedrucktes Sicherheitsmerkmal ist, welches mit einer Druckfarbe enthaltend mindestens ein Pigment gemäß einem oder mehreren der Ansprüche 1 bis 7 gedruckt wird.

16. Sicherheitsanwendung gemäß Anspruch 14, **dadurch gekennzeichnet, dass** das Sicherheitsmerkmal darin besteht, dass das Pigment gemäß einem oder mehreren der Ansprüche 1 bis 7 in das Basismaterial für Papier, Karton, Pappe und/oder Kunststoffe direkt eingearbeitet wird und dort sowie im daraus hergestellten Endprodukt in statistischer Verteilung vorliegt.

17. Sicherheitsanwendung gemäß einem oder mehreren der Ansprüche 14 bis 16, **dadurch gekennzeichnet, dass** das Sicherheitsmerkmal maschinenlesbar ist.

18. Wertdokumente, insbesondere Banknoten, Schecks, Kreditkarten, Aktien, Pässe, Ausweisdokumente, Zutrittsberechtigungsausweise, Führerscheine, Eintrittskarten, Wertmarken, Briefmarken, Etiketten, Siegel, Verpackungsmaterialien, insbesondere Verpackungen von Medikamenten, Lebensmitteln, Parfüms, Zigaretten, oder Produkte des alltäglichen Gebrauchs, insbesondere Bekleidung, Schuhe, Haushaltsartikel, Haushaltselektronikartikel umfassend mindestens ein Sicherheitsmerkmal, welches mindestens ein Pigment gemäß einem oder mehreren der Ansprüche 1 bis 7 enthält.

19. Wertdokumente, insbesondere Banknoten, Schecks, Kreditkarten, Aktien, Pässe, Ausweisdokumente, Zutrittsberechtigungsausweise, Führerscheine, Eintrittskarten, Wertmarken, Briefmarken, Etiketten, Siegel, Verpackungsmaterialien, insbesondere Verpackungen von Medikamenten, Lebensmitteln, Parfüms, Zigaretten, oder Produkte des alltäglichen Gebrauchs, insbesondere Bekleidung, Schuhe, Haushaltsartikel, Haushaltselektronikartikel gemäß Anspruch 18 umfassend mindestens ein weiteres Sicherheitsmerkmal derselben oder anderer Art.

## Claims

1. Pigments comprising a substrate and a coating containing metallic iron, where the substrate comprises titanium oxides, synthetic or natural mica, phyllosilicates, glass, borosilicates, SiO₂, Al₂O₃, graphite and/or BiOCl, the metallic iron is present in the iron-containing coating in combination with FeO and/or Fe₃O₄, and the proportion of metallic iron is 30 to 99% by weight, based on the iron-containing coating.

2. Pigments according to Claim 1, **characterised in that** the proportion of metallic iron in the iron-containing coating is 60 to 85% by weight, based on the iron-containing coating.

3. Pigments according to one or more of Claims 1 to 2, obtainable by reaction of a substrate which has an iron oxide-containing coating as the outermost layer and is optionally additionally coated with one or more layers in a reducing gas mixture comprising nitrogen and hydrogen with formation of metallic iron, and optionally further coating with one or more further layers.

4. Pigments according to one or more of Claims 1 to 3, **characterised in that** the substrate is in flake form.

5. Pigments according to one or more of Claims 1 to 4, **characterised in that** one or more layers comprising metal oxides, metal oxide hydrates, metal suboxides, metals, metal fluorides, metal nitrides, metal oxynitrides and/or mixtures thereof are additionally present between the coating containing metallic iron and the substrate.

6. Pigments according to one or more of Claims 1 to 5, **characterised in that** one or more layers comprising metal oxides, metal oxide hydrates, metal suboxides, metal fluorides, metal nitrides, metal oxynitrides and/or mixtures thereof are additionally present on the coating containing metallic iron.

7. Pigments according to one or more of Claims 1 to 6, **characterised in that** they are furthermore provided with an additional inorganic and/or organic coating as the outer layer.

8. Process for the preparation of pigments according to one or more of Claims 1 to 7, in which a substrate which has an iron oxide-containing coating as the outermost layer and is optionally additionally coated with one or more layers is reacted in a reducing gas mixture comprising nitrogen and hydrogen with formation of metallic iron and is optionally coated with one or more further layers.

9. Process according to Claim 8, **characterised in that** the proportion of hydrogen in the gas mixture comprising nitrogen and hydrogen is 2.5 to 25% by vol.

10. Process according to Claim 8 or 9, **characterised in that** the reduction is carried out at temperatures of 400 to 1000°C.

11. Process according to one or more of Claims 8 to 10, **characterised in that** an inorganic and/or organic coating is additionally applied as the outer layer.

12. Use of pigments according to one or more of Claims 1 to 7 in cosmetics, surface coatings, inks, plastics, films, in security applications, for colouring seed, for colouring foods, for laser marking or in medicament coatings and for the preparation of pigment preparations and dry preparations.

13. Use according to Claim 12, **characterised in that** the pigments according to one or more of Claims 1 to 7 are present in a blend with organic or inorganic dyes and/or pigments.

14. Security application, **characterised in that** documents of value, in particular banknotes, cheques, credit cards, shares, passports, identity documents, access authorisation identity cards, driving licences, entry tickets, revenue stamps, postage stamps, labels, seals, packaging materials, in particular packaging of medicaments, foods, perfumes, cigarettes, or products of daily use, in particular clothing, shoes, household articles, domestic electronic articles, are provided with at least one security feature which contains at least one pigment according to one or more of Claims 1 to 7.

15. Security application according to Claim 14, **characterised in that** the security feature is a printed security feature which is printed using a printing ink comprising at least one pigment according to one or more of Claims 1 to 7.

16. Security application according to Claim 14, **characterised in that** the security feature consists **in that** the pigment according to one or more of Claims 1 to 7 is incorporated directly into the base material for paper, board, cardboard and/or plastics and is in a random distribution therein and in the end product produced therefrom.

17. Security application according to one or more of Claims 14 to 16, **characterised in that** the security feature is machine-readable.

18. Documents of value, in particular banknotes, cheques, credit cards, shares, passports, identity documents, access authorisation identity cards, driving licences, entry tickets, revenue stamps, postage stamps, labels, seals, packaging materials, in particular packaging of medicaments, foods, perfumes, cigarettes, or products of daily use, in particular clothing, shoes, household articles, domestic electronic articles, which include at least one security feature which contains at least one pigment according to one or more of Claims 1 to 7.

19. Documents of value, in particular banknotes, cheques, credit cards, shares, passports, identity documents, access authorisation identity cards, driving licences, entry tickets, revenue stamps, postage stamps, labels, seals, packaging materials, in particular packaging of medicaments, foods, perfumes, cigarettes, or products of daily use, in particular clothing, shoes, household articles, domestic electronic articles, according to Claim 18 which include at least one further security feature of the same or a different type.

## Revendications

1. Pigments comprenant un substrat et un revêtement contenant du fer métallique, dans lesquels le substrat comprend des oxydes de titane, du mica synthétique ou naturel, des phyllosilicates, du verre, des borosilcates, du SiO₂, de l'Al₂O₃, du graphite et/ou du BiOCl, le fer métallique est présent dans le revêtement contenant du fer en combinaison avec du FeO et/ou du Fe₃O₄, et la proportion de fer métallique est de 30 à 99% en poids, sur la base du revêtement contenant du fer.

2. Pigments selon la revendication 1, **caractérisé en ce que** la proportion de fer métallique dans le revêtement contenant du fer est de 60 à 85% en poids, sur la base du revêtement contenant du fer.

3. Pigments selon une ou plusieurs des revendications 1 et 2, pouvant être obtenus par réaction d'un substrat qui comporte un revêtement contenant de l'oxyde de fer en tant que couche la plus externe et qui est en option recouvert de façon additionnelle d'une ou de plusieurs couche(s) dans un mélange de gaz de réduction comprenant de l'azote et de l'hydrogène avec formation de fer métallique, et en option, un autre revêtement muni d'une ou de plusieurs autre(s) couche(s).

4. Pigments selon une ou plusieurs des revendications 1 à 3, **caractérisés en ce que** le substrat est sous forme de flocons.

5. Pigments selon une ou plusieurs des revendications 1 à 4, **caractérisés en ce qu'**une ou plusieurs couche(s) comprenant des oxydes métalliques, des hydrates d'oxydes métalliques, des sous-oxydes de métaux, des métaux, des fluorures de métaux, des nitrures de métaux, des oxynitrures de métaux et/ou des mélanges afférents est/sont de façon additionnelle présente(s) entre le revêtement contenant le fer métallique et le substrat.

6. Pigments selon une ou plusieurs des revendications 1 à 5, **caractérisés en ce qu'**une ou plusieurs couche(s) comprenant des oxydes métalliques, des hydrates d'oxydes métalliques, des sous-oxydes de métaux, des fluorures de métaux, des nitrures de métaux, des oxynitrures de métaux et/ou des mélanges afférents est/sont de façon additionnelle présente(s) sur le revêtement contenant le fer métallique.

7. Pigments selon une ou plusieurs des revendications 1 à 6, **caractérisés en ce qu'**ils sont en outre munis d'un revêtement inorganique et/ou organique additionnel en tant que couche externe.

8. Procédé pour la préparation de pigments selon une ou plusieurs des revendications 1 à 7, dans lequel un substrat qui comporte un revêtement contenant de l'oxyde de fer en tant que couche la plus externe et qui est en option revêtu de façon additionnelle d'une ou de plusieurs couche(s) est amené à réagir dans un mélange de gaz de réduction comprenant de l'azote et de l'hydrogène avec formation de fer métallique et est en option revêtu d'une ou de plusieurs autre(s) couche(s).

9. Procédé selon la revendication 8, **caractérisé en ce que** la proportion d'hydrogène dans le mélange de gaz comprenant de l'azote et de l'hydrogène est de 2,5 à 25% en volume.

10. Procédé selon la revendication 8 ou 9, **caractérisé en ce que** la réduction est mise en oeuvre à des températures de 400 à 1000°C.

11. Procédé selon une ou plusieurs des revendications 8 à 10, **caractérisé en ce qu'**un revêtement inorganique et/ou organique est appliqué de façon additionnelle en tant que couche externe.

12. Utilisation de pigments selon une ou plusieurs des revendications 1 à 7 dans des cosmétiques, des revêtements de surface, des encres, des matières plastiques, des films, dans des applications de sécurité, pour colorer des semences, pour colorer des aliments, pour un marquage laser ou dans des enrobages de médicaments et pour la préparation de préparations de pigments et de préparations sèches.

13. Utilisation selon la revendication 12, **caractérisée en ce que** les pigments selon une ou plusieurs des revendications 1 à 7 sont présents selon un mélange avec des colorants organiques ou inorganiques et/ou des pigments.

14. Application de sécurité, **caractérisée en ce que** des documents de valeur, en particulier des billets de banque, des chèques, des cartes de crédit, des actions, des passeports, des documents d'identité, des cartes d'identité pour autorisation d'accès, des permis de conduire, des tickets d'entrée, des timbres fiscaux, des timbres d'affranchissement, des étiquettes, des scellés, des matériaux de conditionnement, en particulier pour le conditionnement de médicaments, d'aliments, de parfums, de cigarettes, ou de produits d'utilisation quotidienne, en particulier les vêtements, les chaussures, les articles ménagers, les articles électroniques domestiques, sont pourvus d'au moins une caractéristique de sécurité qui contient au moins un pigment selon une ou plusieurs des revendications 1 à 7.

15. Application de sécurité selon la revendication 14, **caractérisée en ce que** la caractéristique de sécurité est une caractéristique de sécurité imprimée qui est imprimée en utilisant une encre d'impression comprenant au moins un pigment selon une ou plusieurs des revendications 1 à 7.

16. Application de sécurité selon la revendication 14, **caractérisée en ce que** la caractéristique de sécurité qui est constituée par le pigment selon une ou plusieurs des revendications 1 à 7 est incorporée directement à l'intérieur du matériau de base pour du papier, du bristol, du carton et/ou des matières plastiques et est selon une distribution aléatoire dedans et dans le produit final produit à partir de ce même matériau de base.

17. Application de sécurité selon une ou plusieurs des revendications 14 à 16, **caractérisée en ce que** la caractéristique de sécurité est lisible par machine.

18. Documents de valeur, en particulier des billets de banque, des chèques, des cartes de crédit, des actions, des passeports, des documents d'identité, des cartes d'identité pour autorisation d'accès, des permis de conduire, des tickets d'entrée, des timbres fiscaux, des timbres d'affranchissement, des étiquettes, des scellés, des matériaux de conditionnement, en particulier pour le conditionnement de médicaments, d'aliments, de parfums, de cigarettes, ou de produits d'utilisation quotidienne, en particulier les vêtements, les chaussures, les articles ménagers, les articles électroniques domestiques, lesquels incluent au moins une caractéristique de sécurité qui contient au moins un pigment selon une ou plusieurs des revendications 1 à 7.

19. Documents de valeur, en particulier des billets de banque, des chèques, des cartes de crédit, des actions, des passeports, des documents d'identité, des cartes d'identité pour autorisation d'accès, des permis de conduire, des tickets d'entrée, des timbres fiscaux, des timbres d'affranchissement, des étiquettes, des scellés, des matériaux de conditionnement, en particulier pour le conditionnement de médicaments, d'aliments, de parfums, de cigarettes, ou de produits d'utilisation quotidienne, en particulier les vêtements, les chaussures, les articles ménagers, les articles électroniques domestiques, selon la revendication 18, lesquels incluent au moins une autre caractéristique de sécurité du même type ou d'un type différent.
